# EUROPEAN PATENT APPLICATION

(11) **EP 2 942 049 A1**
(43) Date of publication of application: **11.11.2015**
(21) Application number: 14167683.3
(22) Date of filing: 09.05.2014
(51) Int. Cl.: A61K 8/46, A61Q 5/06, A61K 8/81, A61K 8/22

(54) **Method of forming a polymer inside the hair shaft comprising using a salt of thiosulfonic acid**

(71) Applicant: The Procter & Gamble Company, Cincinnati, OH 45202 (US)
(72) Inventor: Flohr, Andreas, 64274 Darmstadt (DE); Kripp, Thomas, 64274 Darmstadt (DE)
(74) Representative: Boubel, Thomas

(57) **Abstract**

Method of forming a polymer inside the hair shaft. The polymer changes the mechanical properties of the hair, which provides styling advantages such as improved manageability. The method comprises applying an activating composition to the hair, wherein the activating composition comprises from about 0.5% to about 20% salt of thiosulfonic acid. Also related processes, uses, kits and regimens.

## Description

### FIELD OF THE INVENTION

Method of forming a polymer inside the hair shaft. The polymer changes the mechanical properties of the hair, which provides styling advantages such as improved manageability.

### BACKGROUND OF THE INVENTION

Methods for chemically modifying the internal region of the hair shaft are known in the art. WO2009/088520A, WO2012/100006A, WO2012/100007A and EP2295029A describe the use of ethylenic monomers to chemically modify the internal region of the hair shaft - in particular the ethylenic molecules may bond to the hair and/or to each other to form larger molecules e.g. polymers inside the hair. This increases the rigidity of the hair via the modification of the internal structure of the hair shaft, which provides styling advantages e.g. allowing style formation or increased volume and style retention for longer periods of time. 3-sulfopropyl(meth)acrylate potassium salt (3-SPA) is a monomer for use in chemically modifying the internal region of the hair shaft and has a M.Wt. of 232.3 g/mol. US3,472,243 discloses a process for strengthening and conditioning over-porous hair by polymerizing in said hair a water soluble polymerizable monomeric vinylic compound having acid groups and then bridging the polymer chains so formed with a bridging agent to reduce the water solubility of the polymer chains.

There is a need for improvements in the modification of the internal region of the hair shaft. Whilst certain previous methods may provide satisfactory results to the hair shaft, there is a constant need for providing methods resulting in further improved performance, efficacy, and/or efficiency. There is a need for improving the durability of the treatment such that the benefits last for a longer time and for tailoring the reactivity of the active(s). There is also a need for providing more environmentally friendly and sustainable methods as well as making the method more economically viable and available to a greater breadth of consumer type. In addition there is a need for better ensuring that the results are more predictable and reducing the variability of the end result between consumers with different hair types.

### SUMMARY OF THE INVENTION

According to a first aspect, the present invention relates to a method of forming a polymer inside the hair shaft. The method comprises the steps as specified in the claims and disclosed herein.

According to a second aspect, the present invention relates to a process for demonstrating polymerisation comprising:
(i) forming a demo composition comprising:
   - an ethylenic monomer having a molecular weight of 250 g/mole or less;
   - an radical-forming agent;
   - a dye;
   - cosmetically acceptable carrier;
   - sodium thiosulfate;
      wherein the demo composition exhibits a colour corresponding to the colour of the dye;
(ii) and then observing an increase in viscosity and a decrease in the intensity of the colour.

According to a third aspect, the present invention relates to a regimen for styling hair comprising the method according to the first aspect, wherein the method is repeated at least 2 times at a frequency of at least once every 10 days.

According to a fourth aspect, the present invention relates to a kit comprising:
(i) monomer composition, wherein the monomer composition comprises an ethylenic monomer having a molecular weight of 250 g/mole or less;
(ii) an activating composition, wherein the reducing composition comprises sodium thiosulfate;
(iii)an initiator composition, wherein the initiator composition comprises radical-forming agent.

### BRIEF DESCRIPTION OF THE DRAWINGS

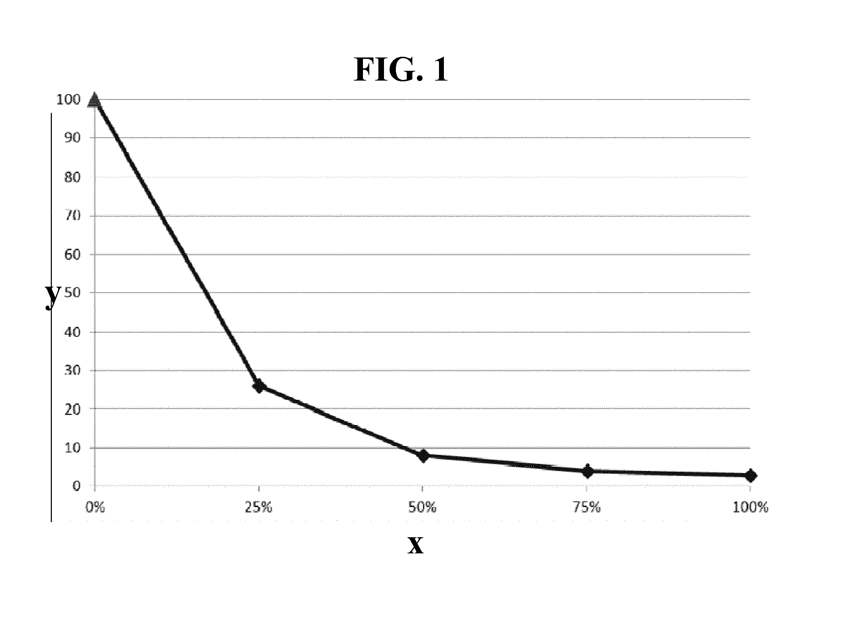

Fig. 1: Shows the correlation between the time (minutes) it takes until start of the polymerisation and the percentage of reducing agent. The x-axis shows amount of reducing agent (X% means X% of a 10% solution of sodium thiosulfate) and the y-axis is time in minutes. NB: with zero sodium thiosulfate (0%) it takes much more than 100 minutes (several weeks!), however, for the sake of a manageable scale factor, this value has been fixed at 100 minutes, but the triangle symbolises that this value of 100 min is much higher.

### DETAILED DESCRIPTION OF THE INVENTION

### General and definitions

In this document, including in all embodiments of all aspects of the present invention, the following definitions apply unless specifically stated otherwise. All percentages are by weight of the total composition. All ratios are weight ratios. References to 'parts' e.g. a mixture of 1 part X and 3 parts Y, is a ratio by weight. "QS" or "QSP" means sufficient quantity for 100% or for 100g. +/- indicates the standard deviation. All ranges are inclusive and combinable. The number of significant digits conveys neither a limitation on the indicated amounts nor on the accuracy of the measurements. All numerical amounts are understood to be modified by the word "about". All measurements are understood to be made at 23°C and at ambient conditions, where "ambient conditions" means at 1 atmosphere (atm) of pressure and at 50% relative humidity. "Relative humidity" refers to the ratio (stated as a percent) of the moisture content of air compared to the saturated moisture level at the same temperature and pressure. Relative humidity can be measured with a hygrometer, in particular with a probe hygrometer from VWR® International. Herein "min" means "minute" or "minutes". Herein "mol" means mole. Herein "g" following a number means "gram" or "grams". All weights as they pertain to listed ingredients are based on the active level and do not include carriers or by-products that may be included in commercially available materials. Herein, "comprising" means that other steps and other ingredients can be in addition. "Comprising" encompasses the terms "consisting of" and "consisting essentially of". The compositions, formulations, methods, uses, kits, and processes of the present invention can comprise, consist of, and consist essentially of the elements and limitations of the invention described herein, as well as any of the additional or optional ingredients, components, steps, or limitations described herein. Embodiments and aspects described herein may comprise or be combinable with elements, features or components of other embodiments and/or aspects despite not being expressly exemplified in combination, unless an incompatibility is stated. "In at least one embodiment" means that one or more embodiments, optionally all embodiments or a large subset of embodiments, of the present invention has/have the subsequently described feature.

"Molecular weight" or "M.Wt." or "MW" and grammatical equivalents mean the number average molecular weight.

"Viscosity" is measured at 25°C using a HAAKE Rotation Viscometer VT 550 with cooling/heating vessel and sensor systems according to DIN 53019 at a shear rate of 12.9 s⁻¹.

"Water-soluble" refers to any material that is sufficiently soluble in water to form a clear solution to the naked eye at a concentration of 0.1% by weight of the material in water at 25°C. The term "water-insoluble" refers to any material that is not "water-soluble".

"Dry" or "substantially dry" means comprising less than 5%, less than 3% or, less than 2%, less than 1%, or about 0% of any compound or composition being in liquid form when measured at 25°C at ambient conditions. Such compounds or compositions being in liquid form include water, oils, organic solvents and other wetting agents. "Anhydrous" means that the composition comprises less than 5%, less than 3% or, less than 2%, less than 1%, or about 0% water by total weight of the composition.

"Substantially free from" or "substantially free of" means less than about 1%, or less than 0.8%, or less than 0.5%, or less than 0.3%, or about 0%, by total weight of the composition or formulation.

"Hair" means mammalian keratin fibres including scalp hair, facial hair and body hair. It includes such hair still being attached to a living subject and also hair that has been removed therefrom such as hair swatches and hair on a doll/mannequin. In at least one embodiment, "hair" means human hair. "Hair shaft" or "hair fibre" means an individual hair strand and may be used interchangeably with the term "hair."

"Proximal to the scalp" means that portion of an extended, or substantially straightened, hair shaft that is closer in distance to the scalp than to the end of the hair. Thus, about 50% of the hair fibre length would be considered proximal to the scalp, and about 50% of the hair fibre would be distal to the scalp. "z cm proximal to the scalp" means a distance "z" along the hair, with one endpoint being on or directly adjacent to the scalp, and the second endpoint being measured "z" centimetres along the length of the extended or substantially straightened hair.

"Chemically modify" or grammatical equivalents thereof, means that a chemical moiety such as monomer and/or crosslinker and/or polymer, stably affixes to a second chemical moiety, for example, a keratin protein, another component of hair, and/or another monomer or crosslinker or polymer. Normally, "chemically modify" means stably affix via a covalent bond, unless otherwise stated.

"Cosmetically acceptable" means that the compositions, formulations or components described are suitable for use in contact with human keratinous tissue without undue toxicity, incompatibility, instability, allergic response, and the like. All compositions and formulations described herein which have the purpose of being directly applied to keratinous tissue are limited to those being cosmetically acceptable.

"Derivatives" includes but is not limited to, amide, ether, ester, amino, carboxyl, acetyl, acid, salt and/or alcohol derivatives of a given compound. In at least one embodiment, "derivatives thereof" means the amide, ether, ester, amino, carboxyl, acetyl, acid, salt and alcohol derivatives.

"Monomer" means a discrete, non-polymerised chemical moiety capable of undergoing polymerisation in the presence of an initiator or any suitable reaction that creates a macromolecule e.g. such as polycondensation, polyaddition, anionic or cationic polymerization. "Unit" means a monomer that has already been polymerised i.e. is part of a polymer.

"Polymer" means a chemical formed from the polymerisation of two or more monomers. The term "polymer" shall include all materials made by the polymerisation of monomers as well as natural polymers. Polymers made from only one type of monomer are called homopolymers. Herein, a polymer comprises at least two monomers. Polymers made from two or more different types of monomers are called copolymers. The distribution of the different monomers can be random, alternating or block-wise (i.e. block copolymer). The term "polymer" used herein includes any type of polymer including homopolymers and copolymers.

"Kit" means a package comprising a plurality of components. "Kit" may be referred to as "kit-of-parts". An example of a kit is, for example, a first composition and a separately packaged second composition and optionally application instructions.

### Theory behind and advantages of the invention

As already discussed above, there is a constant need for improvements in the modification of the hair shaft. The invention described herein provides significant improvements versus available technologies. Available technologies on polymerisation inside the hair deal with two main active ingredients: an ethylenic monomer and a radical-forming agent such as hydrogen peroxide. The peroxide creates radicals which initiate the polymerisation process. However, hydrogen peroxide is rather stable in 'clean' conditions i.e. without the presence of a proper redox partner. So, after combining those main active ingredients, the time until the polymerisation starts depends on the health/history/quality of the particular hair fibre(s) and so it is hard to predict exact polymerisation start in view the variability in hair fibre(s) across the human population. The present invention has the advantage that start time of polymerisation is defined. Thus the present invention solves the problem of predictability of treatments for methods of forming a polymer inside the hair shaft. For example, after a dwell time of ethylenic monomer plus radical-forming agent on the hair, the polymerisation can be initiated by adding an activating agent, such as sodium thiosulfate. High concentrations of activating agent lead to a more or less immediate polymerisation, whereas lower concentrations may provide a certain delay thereafter, see Fig. 1.

The aspects of the invention and a description of the features and embodiments thereof are described in more detail hereinafter.

### Method

The present invention relates to a method of forming a polymer inside the hair shaft. In at least one embodiment, the method relates to a method of forming a polymer inside the hair shaft, the method comprising: (a) applying an initiator composition to the hair, wherein the initiator composition comprises radical-forming agent and cosmetically acceptable carrier; then (b) allowing the initiator composition to remain on the hair for at least 2 min; then (c) applying a monomer composition to the hair, wherein the monomer composition comprises an ethylenic monomer having a molecular weight of 250 g/mole or less; then (d) allowing the monomer composition to remain on the hair for at least 2 min; then (e) applying an activating composition to the hair, wherein the activating composition comprises from about 0.5% to about 20% salt of thiosulfonic acid; then (f) allowing the activating composition to remain on the hair for at least 2 min; then (g) rinsing the hair.

Alternatively, the method comprises: (a) applying a monomer composition to the hair, wherein the monomer composition comprises an ethylenic monomer having a molecular weight of 250 g/mole or less; then (b) allowing the monomer composition to remain on the hair for at least 2 min; then (c) applying an initiator composition to the hair, wherein the initiator composition comprises radical-forming agent and cosmetically acceptable carrier; then (d) allowing the initiator composition to remain on the hair for at least 2 min; then (e) applying an activating composition to the hair, wherein the activating composition comprises from about 0.5% to about 20% salt of thiosulfonic acid; then (f) allowing the activating composition to remain on the hair for at least 2 min; then (g) rinsing the hair.

Alternatively, the method comprises: (a) applying a monomer composition to the hair, wherein the monomer composition comprises an ethylenic monomer having a molecular weight of 250 g/mole or less; then (b) allowing the monomer composition to remain on the hair for at least 2 min; then (c) applying an activating composition to the hair, wherein the activating composition comprises from about 0.5% to about 20% salt of thiosulfonic acid; then (d) allowing the activating composition to remain on the hair for at least 2 min; then (e) applying an initiator composition to the hair, wherein the initiator composition comprises radical-forming agent and cosmetically acceptable carrier; then (f) allowing the initiator composition to remain on the hair for at least 2 min; then (g) rinsing the hair.

Alternatively, the method comprises: (a) applying to hair: an initiator composition, wherein the initiator composition comprises radical-forming agent; and a monomer composition to the hair, wherein the monomer composition comprises an ethylenic monomer having a molecular weight of 250 g/mole or less; and a cosmetically acceptable carrier; (b) and subsequently allowing the compositions, which may be premixed before application, to remain on the hair for a period of time w, wherein time w is from about 2 min to about 45 min, or from about 3 min to about 20 min, or from about 4 min to about 10 min; then (c) applying an activating composition to the hair, wherein the activating composition comprises from about 0.5% to about 20% salt of thiosulfonic acid; and (d) allowing the activating composition to remain on the hair for at least 2 min; (e) and subsequently rinsing the hair.

Alternatively, the method comprises: (a) applying to hair: activating composition, wherein the activating composition comprises from about 0.5% to about 20% salt of thiosulfonic acid; and a monomer composition to the hair, wherein the monomer composition comprises an ethylenic monomer having a molecular weight of 250 g/mole or less; and a cosmetically acceptable carrier; (b) and subsequently allowing the compositions, which may be premixed before application, to remain on the hair for a period of time w, wherein time w is from about 2 min to about 45 min, or from about 3 min to about 20 min, or from about 4 min to about 10 min; then (c) applying an initiator composition to the hair, wherein the initiator composition comprises radical-forming agent; and then (d) allowing the activating composition to remain on the hair for at least 2 min; (e) and subsequently rinsing the hair.

In at least one embodiment, inside the hair shaft means the internal region of the hair shaft. In at least one embodiment, the method relates to the modification of the internal region of the hair shaft with a monomer and/or polymer; and combinations thereof. In at least one embodiment, the method relates to the forming of a polymer in the internal region of the hair shaft by *in situ* polymerisation, wherein the polymerisation that occurs is free radical polymerisation.

In at least one embodiment, after applying the initiator composition to the hair but the subsequent step, the initiator composition is allowed to remain on the hair for a period of time y, wherein time y is from about 2 min to about 120 min, or from about 2 min to about 45 min, or from about 3 min to about 20 min, or from about 4 min to about 10 min.

In at least one embodiment, the method comprises the step of de-wetting the hair, which occurs after application of the initiator composition and prior to the application of any further composition such as the monomer composition. In at least one embodiment, the de-wetting the hair comprises the application of an absorbent material to the hair such that wetness is transferred from the hair to the absorbent material and wherein the wetness comprises the cosmetically acceptable carrier. The absorbent material may be selected from the group consisting of: towel, absorbent paper, and combinations thereof. In at least one embodiment, the de-wetting the hair comprises allowing moisture to evaporate from the hair wherein the moisture comprises the cosmetically acceptable carrier. In at least one embodiment, the de-wetting the hair comprises towel drying the hair such that the initiator composition no longer drips from the hair. In at least one embodiment, the de-wetting the hair comprises removing superficial initiator composition from the hair. In at least one embodiment, the de-wetting the hair does not comprise rinsing the initiator composition from the hair. The de-wetting the hair may last for time z, wherein time z is from about 1 min to about 120 min, or from about 2 min to about 45 min, or from about 3 min to about 20 min, or from about 4 min to about 10 min.

In at least one embodiment, the method comprises allowing the monomer composition to remain on the hair for a period of time x, wherein the time x is from about 2 min to about 120 min; and then rinsing the hair; and then washing the hair. The time x may be from about 5 min to about 100 min, or from about 10 min to about 90 min, or from about 20 min to about 60 min.

In at least one embodiment, the method comprises the application of an energy delivery device to the hair, which may be applied proximal to the scalp. In at least one embodiment, the energy is heat.

In at least one embodiment, the method comprises exposing the hair to a relative humidity (RH) of at least about 70%, within 1 hour of applying the monomer composition, or activating composition, or activated composition, and said exposure lasting from about 2 min to about 90 min. Indeed, the application of high RH has numerous benefits with regard to this technology. It is believed that exposing the hair to high RH during the chemical modification of the internal region of a hair shaft improves the diffusion of the monomers through the hair and/or increases the penetration of the monomers into the hair shaft. More specifically, it is believed that components of the treatment compositions are maintained in solution for longer and are more mobile. Furthermore, high RH is thought to result in a swollen hair shaft and a more open cuticle. In at least one embodiment, the hair is exposed to the relative humidity by using a device in the vicinity of the hair, preferably less than 0.5 m from the hair, more preferably 1 cm to 30 cm from the hair. In at least one embodiment, the device is a water vapour-imparting device, or wherein water vapour-imparting device is an electronic hood-shaped device. Such device is particularly suitable for improving the performance, efficacy and/or efficiency of the method. Suitable devices may include: Hairspa ION, by Wella, Darmstadt, Germany; Electronic Master Ionic Action, by Müster; Blitz Super Electronic Ozono, by Ceriotti; Beautivap Digital Ozon and Ozono Energy, by Artem; Mega Ozono, by MediaLine; Mod. 370, by bmp; Steam Machine, by REM; Micro Mist (SD200NIW) and Belmaster (BM-975), by Takara Belmont. A suitable device is described in EP1871194.

The rinsing step in the methods of the present invention may last from about 10 sec to about 15 min.

In at least one embodiment, the method comprises drying the hair, or drying the hair by using a towel and/or blow dryer.

In at least one embodiment, the method does not comprise applying to the hair any of the following: transition metal, alcohol, ammonia, lactone compound, mercuric compound, silicone compound, fatty ester, fatty alcohol, hydrocarbon comprising 14 or more carbon atoms.

### Initiator composition

In at least one embodiment, the method comprises applying an initiator composition to the hair. The initiator composition comprises radical-forming agent. In at least one embodiment, initiator composition is in solid form. In at least one embodiment, the solid form is selected from the group consisting of: powder, granules, and combinations thereof. In an embodiment, the initiator composition is substantially free of oily compounds. In an embodiment, the initiator formulation comprises a salt.

In at least one embodiment, the radical-forming agent is selected from the group consisting of: peroxides, for example hydrogen peroxide; persulfates, for example potassium persulfate or sodium persulfate; and mixtures thereof. In at least one embodiment, the radical-forming agent is a peroxide, and the peroxide is present in an amount of from about 0.5% to about 5%, or from about 1% to about 4%, or from about 1.3% to about 3%, or from about 1.5% to about 3%. In at least one embodiment, the initiator composition is substantially free of hair colouring agents, or substantially free of oxidative dyes and/or direct dyes. In at least one embodiment, the radical-forming agent is hydrogen peroxide. In at least one embodiment, the initiator composition comprises from about 0.01% to about 20%, or from about 0.1% to about 15%, or from about 1% to about 10%, or from about 1.5% to about 6.5%, or from about 2% to about 3% radical-forming agent.

In at least one embodiment, the initiator composition comprises from about 2% to about 3% hydrogen peroxide. Hydrogen peroxide is useful in view of being efficacious as a radical-forming agent, easily and relatively economically to procure. In at least one embodiment, the initiator composition comprises from about 2% to about 3% hydrogen peroxide, and wherein the hydrogen peroxide is the sole radical-forming agent in the initiator composition.

In at least one embodiment, the initiator composition comprises a buffer system for stabilising the pH. Suitable buffers may also act as chelating agents. Chelation of transition metals, for example copper or iron from pipes which might be present in trace amounts in tap water, is important because peroxides, for example, are sensitive to cleavage by transition metals. In the absence of a buffer system the transition metal may cleave the peroxide, deactivating it. Typical buffer systems comprise a strong acid and its weak conjugate base or a weak base and its conjugate acid. In at least one embodiment, the initiator composition comprises a buffer system wherein the buffer system is phosphoric acid and disodium phosphate. Another example of a suitable buffer system is citric acid and sodium hydroxide.

In at least one embodiment, the initiator composition has a pH of from about pH 2 to about pH 10, or from about pH 4 to about pH 8, or from about pH 5 to about pH 7.

In at least one embodiment, the initiator composition is substantially free of inorganic cation, for example inorganic cations having a charge of at least 2+, or at least 3+. In at least one embodiment, the initiator composition is substantially free of inorganic cations and transition metals.

### Monomer composition

The monomer composition comprises an ethylenic monomer having a molecular weight of 250 g/mole or less. In at least one embodiment, the ethylenic monomer comprises a vinyl group. "Vinyl group" as used herein, means H₂C=CH-R. In at least one embodiment, the ethylenic monomer comprises an acrylate group or a methacrylate group. "Acrylate group" as used herein, means H₂C=CH-C(O)O-R. Acrylic acid, for example, comprises an acrylate group since R is hydrogen. "Methacrylate group" as used herein means H₂C=C(CH₃)-C(O)O-R.

In at least one embodiment, the monomer composition comprises an ethylenic monomer selected from the group consisting of: mesaconic acid, 2-pentenoic acid, tiglic acid, tiglic acid esters, furan-3-acrylic acid, 2-acrylamido-2-methyl-1-propanesulfonic acid, maleamic acid, 3-aminocrotonic acid, crotonic acid esters, itaconic anhydride, trimethylsilylacrylate, poly(ethyleneglycol)acrylates, N-vinylacetamide, 2-acetamidoacrylic acid, vinylsulfonic acid, tetrahydrofurfurylacrylate, N-methyl-N-vinylacetamide, vinylpropionate, vinylanisole, vinylcrotonate, methyl 3-hydroxy-2-methylenebutyrate, methacryloyl-L-lysine, N-(2-hydroxypropyl)methacrylamide, 2-acrylamidodiglycolic acid, 2-ethoxyethyl acrylate, 2-butoxyethyl acrylate, N-isopropylmethacryalmide, 2-aminoethyl methacrylate, 2-bromoethyl acrylate, 3-(dimethylamino)propyl acrylate, (3-acrylamidopropyl)trimethyl ammonium salt, [2-(acryloyloxy)ethyl]-trimethylammonium salt, alkylacetamidoacrylate, sulfoalkyl (meth)acrylate, 3-sulfopropyl acrylate, 3-sulfopropyl methacrylate, derivatives thereof, and mixtures thereof. In at least one embodiment, the composition comprises at least one ethylenic monomer, selected from the group of compounds listed above, as the sole ethylenic monomer(s). In at least one embodiment, the monomer composition comprises an ethylenic monomer selected from the group consisting of: selected from the group consisting of 3-sulfopropyl acrylate, 3-sulfopropyl methacrylate, and salts, derivatives and mixtures thereof. In at least one embodiment, the sole ethylenic monomer(s) present are selected from the group consisting of 3-sulfopropyl acrylate, 3-sulfopropyl methacrylate, and salts, derivatives thereof, and mixtures thereof. In at least one embodiment, the sole ethylenic monomer is 3-sulfopropyl acrylate. In at least one embodiment, the ethylenic monomer is 3-sulfopropyl acrylate, which is added to the composition as 3-sulfopropyl acrylate potassium salt.

In at least one embodiment, the monomer composition comprises an ethylenic monomer selected from the group consisting of: methacrylic acid, tiglic acid, acrylic acid, and mixtures thereof. In at least one embodiment, the monomer composition comprises at least one ethylenic monomer, selected from the group cited above, as the sole ethylenic monomer(s). In at least one embodiment, the ethylenic monomer is selected from the group consisting of: methacrylic acid, tiglic acid, acrylic acid, 3-sulfopropyl acrylate, 3-sulfopropyl methacrylate, salts thereof, and mixtures thereof; preferably acrylic acid. In at least one embodiment, the sole ethylenic monomer is acrylic acid. The M.Wt. of the ethylenic monomer is important because of the need for the monomer to penetrate into the hair shaft prior to polymerisation. Large and/or bulky monomers would penetrate less easily into the hair shaft. In at least one embodiment, the ethylenic monomer has a M.Wt. of from about 50 g/mole to about 105 g/mole. In at least one embodiment, the ethylenic monomer has a M.Wt. of from about 70 g/mole to about 100 g/mole, or from about 75 g/mol to about 95 g/mol, or from about 75 g/mole to about 90 g/mole. In at least one embodiment, the monomer composition is substantially free of an ethylenic monomer that has a M.Wt. of below about 50 g/mole, or below about 60 g/mol, or above about 200 g/mol, or above about 500 g/mole. In at least one embodiment, the monomer composition comprises from about 1.2%, or 1.3%, or 1.4%, or 1.6%, or 1.8%, or 2.0%, or 2.2%, or 2.4%, or 2.5% to about 4.8%, or 4.7%, or 4.6%, or 4.5%, or 4.3%, or 4.1%, or 4.0%, or 3.9%, or 3.8%, or 3.7%, or 3.6%, or 3.5% ethylenic monomer. This amount may be the total amount of ethylenic monomer in the monomer composition. In at least one embodiment, the sole ethylenic monomer is acrylic acid and the monomer composition comprises from about 3.0% to about 4.2% ethylenic monomer i.e. from about 3.0% to about 4.2% acrylic acid. In at least one embodiment, two or more different ethylenic monomers are present in the monomer composition. The resultant polymers may be copolymers.

In at least one embodiment, the monomer composition is substantially free of any nitrogen-containing ethylenic monomers. In at least one embodiment, the monomer composition is free of a nitrogen-containing ethylenic monomer. In at least one embodiment, the nitrogen-containing ethylenic monomer is selected from the group consisting of: amides, cyanates, nitriles, and ammonium-group containing compounds. In at least one embodiment, the ethylenic monomer is an acid. In at least one embodiment, the ethylenic monomer is not a carboxylic acid. In at least one embodiment, the monomer composition is substantially free of any halogen-containing ethylenic monomers.

In at least one embodiment, the monomer composition comprises an ethylenic monomer selected from the group consisting of: acrylic acid, sodium acrylate, potassium acrylate, calcium acrylate, monoethanolamine acrylate, 3-hydroxypropyl acrylate, 2,5-butylaminoethyl acrylate, methacrylic acid, sodium methacrylate, potassium methacrylate, calcium methacrylate, monoethanolamine methacrylate, 2-N,N-dimethylaminoethyl acrylate, glycidyl methacrylate, 2-dimethylamino ethyl methacrylate, 2-hydroxypropyl methacrylate, 3-hydroxypropyl methacrylate, 2,4-dihydroxybutyl methacrylate, 2,3-epoxybutyl methacrylate, 2-t-butylaminoethyl methacrylate, 2-(2-diethylamino)ethyl methacrylate, ethylene glycol mono methacrylate, itaconic acid (and salts thereof), vinyl pyridine, resorcinol, and mixtures thereof.

In at least one embodiment, the monomer composition comprises an inhibitor compound selected from the group consisting of: 2-*tert*-butyl-4-hydroxy-anisole, 3-*tert*-butyl-4-hydroxyanisole, and mixtures thereof. The inhibitor compound may be present in the monomer composition in an amount of from about 1 milligram to about 1000 milligram per kilogram of the ethylenic monomer. Such amount may be the total amount of inhibitor compound being those selected from the group consisting of: 2-*tert*-butyl-4-hydroxy-anisole, 3-*tert*-butyl-4-hydroxyanisole. In at least one embodiment, the inhibitor compound is present in the monomer composition in an amount of from about 50 milligram to about 800 milligram, or from about 100 milligram to about 500 milligram, or from about 100 milligram to about 300 milligram, or from about 100 milligram to about 200 milligram, per kilogram of ethylenic monomer. In at least one embodiment, the inhibitor compound is the mixture of 2-*tert*-butyl-4-hydroxy-anisole and 3-*tert-*butyl-4-hydroxy-anisole. In at least one embodiment, the mixture of 2-*tert*-butyl-4-hydroxyanisole and 3-*tert*-butyl-4-hydroxy-anisole comprises greater than about 50%, or greater than about 60%, or greater than about 80% 3-*tert*-butyl-4-hydroxy-anisole. The inhibitor compound functions as a polymerisation inhibitor i.e. it stabilises the ethylenic monomer so that it does not start to polymerise prior to contact with radical-forming agent etc. The inhibitor compound provides excellent performance in that the ethylenic monomer is effectively stabilised and also when the ethylenic monomer exposed to radical-forming agent etc, the ethylenic monomer can polymerises efficiently and quickly. In at least one embodiment, the monomer composition is substantially free of a polymer derived from the polymerisation of ethylenic monomers. Other polymerisation inhibitors used for stabilising ethylenic monomers are known in the art e.g. 4-methoxy phenol. In at least one embodiment, the monomer composition comprises less than about 500 ppm, or less than about 400 ppm, or less than about 300 ppm, or less than about 200 ppm, or less than about 100 ppm, or less than about 50 ppm, or less than about 10 ppm, of 4-methoxy phenol. In at least one embodiment, the monomer composition is substantially free of a polymerisation inhibitor, with the exception of an inhibitor compound selected from the group consisting of: 2-*tert*-butyl-4-hydroxy-anisole and 3-*tert*-butyl-4-hydroxy-anisole.

The monomer composition may comprise a crosslinker having a M.Wt. suitable to penetrate the hair shaft. In at least one embodiment, the monomer composition is substantially free of crosslinker.

The monomer composition may comprise a viscosity-increasing agent. Viscosity is important when the monomer composition is in the form of gel, cream, lotion, emulsion and the like because it prevents the monomer composition from sliding off the skin and/or hair. However, lower viscosities allow actives to penetrate/diffuse more easily e.g. into the internal region of the hair shaft. The viscosity of the monomer composition when it is in the form of a gel may be from about 500 mPa·s to about 15000 mPa·s, or from about 1000 mPa·s to about 10000 mPa·s, or from about 1500 mPa·s to about 7500 mPa·s, or from about 2000 mPa·s to about 5000 mPa·s, measured with a Brookfield Viscosimeter RVDV III Ultra CP 52 at 25 °C and 1 rpm. The viscosity-increasing agent may be selected from the group consisting of non-ionic thickeners, cationic thickeners, anionic thickeners, amphoteric thickeners, and mixtures thereof. The viscosity-increasing agent may be present in the monomer composition in an amount of from about 0.1% to about 10%, or from about 0.2% to about 5.0%. In at least one embodiment, the monomer composition comprises a non-ionic or anionic thickener (or mixtures thereof), which is in view of the typically anionic chemistry of any polymerised ethylenic monomer. Non-ionic or anionic thickeners are less likely to interact directly with any formed polymer and hence the formation of insoluble complexes or precipitates is also less likely. In at least one embodiment, the viscosity-increasing agent is stable at the required pH and does not substantially affect the active levels of ethylenic monomer. The viscosity-increasing agent may be a cross-linked or a non-crosslinked polymer. In at least one embodiment, the viscosity-increasing agent is a hydrophobically-modified polyacrylate polymer. The monomer composition may comprise from about 0.5% to about 1.5% of the hydrophobically-modified polyacrylate polymer, by total weight of the monomer composition. Suitable hydrophobically-modified polyacrylate polymers include: acrylates/C10-C30 alkylacrylates copolymers such as Ultrez^{®} 20/21 from Lubrizol, and Permulen^{®} TR1 from Lubrizol; acrylates/beheneth-25 methacrylate copolymers such as Aculyn^{®} 28 from Rohm & Haas; acrylates/ceteth-20 itaconate copolymers such as Structure^{®} 3001 or 2001 from Akzo Nobel. In at least one embodiment, the viscosity-increasing agent is a non-crosslinked associative thickening polymer. The monomer composition and/or initiator composition may comprise from about 0.5% to about 3% of the non-crosslinked associative thickening polymer, by total weight of the composition. Suitable associative thickeners include polyurethane-based polymers such as polyurethane-30 e.g. LuvigelSTAR^{®} from BASF. Also EO-PO-block copolymers may be useful, for example Pluronics^{®} from BASF. In at least one embodiment, the viscosity-increasing agent comprises at least one polysaccharide. In at least one embodiment, the monomer composition comprises a heteropolysaccharide. The total polysaccharide present in the monomer composition may be from about 0.2% to about 5%, or from about 0.5% to about 4%, by total weight of the monomer composition. Suitable polysaccharides and heterosaccharides include starches and derivatives thereof, e.g. mono- or diesters with phosphoric acid, cellulose types and their derivatives, xanthan gums, carrageenans. Preferred heteropolysaccharides include xanthan gum such as Keltrol^{®} T from Kelco, and Natrosol^{®} 250 HHR from Herkules. In at least one embodiment, the polysaccharide is selected from the group consisting of hydroxyethylcellulose, hydroxypropylcellulose, xanthan gum, carrageenans, and mixtures thereof. Xanthan gums and derivatives thereof may be present in an amount of from about 0.2% to about 1.5%, or from about 0.5% to about 0.9%, by total weight of the monomer composition. Starches and derivatives thereof may be present in an amount of from about 3% to about 4% by total weight of the monomer composition. In at least one embodiment, the viscosity-increasing agent is a starch compound. A suitable starch compound is hydroxypropyl starch phosphate such as Structure^{®} XL from National Starch. In at least one embodiment, the monomer composition comprises two different polysaccharide viscosity-increasing agents.

In at least one embodiment, monomer composition comprises a cosmetically acceptable carrier. In at least one embodiment, the monomer composition comprises at least about 60%, or at least about 70%, or from about 75% to about 95%, or from about 80% to about 90% cosmetically acceptable carrier. The carrier may comprise water. In at least one embodiment, the carrier comprises water, fatty alcohols, volatile organic alcohols, and mixtures thereof. In at least one embodiment, the carrier is water.

In at least one embodiment, the monomer composition is substantially free of: a lactone or an alpha-methylene lactone compound. In at least one embodiment, the monomer composition is substantially free of a mercuric compound. In at least one embodiment, the monomer composition comprises about 5% or less of or is substantially free of at least one of or all of the following: reducing agent, transition metal, alcohol, ammonia, lactone compound, mercuric compound, plasticizer, surfactant, neutralizing agent, propellant, silicone compound, fatty ester, fatty alcohol, hydrocarbon comprising 14 or more carbon atoms, lanolin, vitamin, protein, preservative, dye, tint, bleach, colorant, sunscreen, gelling agent, anti-dandruff active, hair growth active, non-polymeric thickener including clay, perfume. In at least one embodiment, the monomer composition comprises about 5% or less of or is substantially free of at least one or all of the following: reducing agent, transition metal, alcohol, ammonia, lactone compound, mercuric compound, plasticizer, surfactant, neutralizing agent, propellant, silicone compound, fatty ester, fatty alcohol, hydrocarbon comprising 14 or more carbon atoms. Oily compounds may aid the penetration of the ethylenic monomer into the skin and/or scalp, which may not be preferred. In at least one embodiment, the monomer composition is substantially free of any oily compounds. In an embodiment, the monomer composition is substantially free of any of 2-pyrolidinoethanol, 1-piperidine-ethanol, 4-methylmorpholine, 2-morpholinoethanol, tetramethylethylenediamine, salts and/or hydrates of cerium, cobalt, manganese, iron, nickel, copper, and combinations thereof. In an embodiment, the monomer composition is substantially free of any of sodium hydroxide, potassium hydroxide, sodium metasilicate, ammonium hydroxide, ethanolamine, aminomethylpropanol, ammonium carbonate.

In at least one embodiment, the monomer composition has a pH of from about 2 to about 10. In at least one embodiment, the monomer composition has a pH of from about 2.6 to about 4.5. The pH of the cosmetic composition is important because of potential sensitisation of the scalp and potential damage to the hair. Indeed, a pH lower than about 2.6 is not recommended for the present invention. Thus the lower limit of pH 2.6 has the benefit of reduced scalp sensitisation and reduced hair damage compared to a pH lower than this. In at least one embodiment, the pH is from about 2.7 to about 4.0, or from about 2.9 to about 3.9, or from about 3.0 to about 3.8, or from about 3.2 to about 3.7, or from about 3.4 to about 3.6. In at least one embodiment, the pH is from about 2.7 to about 3.8. In at least one embodiment, the monomer composition does not exhibit an alkaline pH. In at least one embodiment, the monomer composition comprises a buffer system. In at least one embodiment, the buffer system comprises an acid and its conjugate base. In at least one embodiment, the buffer system is a phosphate-based buffer system. In at least one embodiment, the acid of the buffer system is phosphoric acid. In at least one embodiment, the buffer system is phosphoric acid (H₃PO₄) and the salt of a hydrogen phosphate ion. In at least one embodiment, the buffer system is citric acid and sodium hydroxide.

In at least one embodiment, the monomer composition comprises an oxygen scavenger. Oxygen scavenger may increase the rate of conversion from monomer into polymer. In at least one embodiment, the oxygen scavenger is ascorbic acid.

In at least one embodiment, the monomer composition comprises: a) from about 2.5% to about 3.5% ethylenic monomer, wherein the ethylenic monomer is selected from the group consisting of: methacrylic acid, acrylic acid, and mixtures thereof; b) a cosmetically acceptable carrier; and wherein the composition has a pH of from about 2.7 to about 3.8; and wherein monomer composition comprises a buffer system; and wherein the monomer composition comprises an inhibitor compound selected from the group consisting of: 2-*tert*-butyl-4-hydroxyanisole, 3-*tert*-butyl-4-hydroxy-anisole, and mixtures thereof.

In at least one embodiment, the monomer composition is substantially free of inorganic cation, for example inorganic cations having a charge of at least 2+, or at least 3+. In at least one embodiment, the monomer composition is substantially free of inorganic cations and transition metals.

### Activating composition

The method comprises applying an activating composition to the hair. The activating composition comprises salt of thiosulfonic acid. The activating composition comprises from about 0.5% to about 20% salt of thiosulfonic acid. In at least one embodiment, the activating composition comprises from about 1% to about 15%, or from about 2% to about 12%, or from about 5% to about 10% salt of thiosulfonic acid. In at least one embodiment, the salt of thiosulfonic acid is sodium thiosulfate. Sodium thiosulfate is advantageous because it has a highly acceptable odour and is an efficacious activating agent. In at least one embodiment, the activating composition comprises from about 1% to about 12%, or from about 4% to about 10%, or from about 8% to about 10% sodium thiosulfate. In at least one embodiment, the sole salt of thiosulfonic acid is sodium thiosulfate. In at least one embodiment, the activating composition comprises from about 8% to about 10% sodium thiosulfate, and wherein the composition is substantially free of any other reducing agent.

Certain reducing agents can be used as a replacement for the salt of thiosulfonic acid, for example: (powdered) sodium metabisulfite, ammonium sulfite, thioglycolic acid, thiolactic acid, ammonium thiolactate, glyceryl monothioglycolate, ammonium thioglycolate, thioglycerol, 2,5-dihydroxybenzoic acid, calcium thioglycolate, isooctyl thioglycolate, D/L-cysteine, monoethanolamine thioglycolate, ascorbic acid, ascoryl palmitate, ascobyl phosphate, and mixtures thereof. Particular reducing agents are: cysteine, ascorbic acid, and mixtures thereof. However, all these reducing agents have the disadvantage that they have poor odour and/or poor performance.

In at least one embodiment, the activating composition has a pH of from about pH 6 to about pH 11, or from about pH 7 to about pH 9. In at least one embodiment, the activating composition comprises a buffer system for stabilising the pH.

### Finishing composition

In at least one embodiment, the method comprising applying a finishing composition to the hair. The finishing composition is useful in that it can provide extra benefits to the hair, such as conditioning or hold, that synergise the effect of the polymerisation inside the hair shaft. Indeed, extra benefits in addition to increased hair manageability (provided by the polymer inside the hair shaft) are highly welcomed by consumers. In at least one embodiment, the finishing composition comprises a conditioning agent. In at least one embodiment, the method comprises applying a finishing composition to the hair, wherein the finishing composition comprises a hydrophobic polyol and a hydrophilic polyol. Employment of a hydrophobic polyol in combination with a hydrophilic polyol provides reduced static advantages for the consumer. In this regard, EP2679215, which published as an A1 spec on 1^{st} Jan 2014 is incorporated herein by reference, particularly the disclosure vis-à-vis the "conditioning composition" from §0052 to §0073. In at least one embodiment, the finishing composition comprises cationic surfactant. In at least one embodiment, the finishing composition comprises from about 0.1% to about 10%, from about 0.25% to about 8%, from about 0.5% to about 5%, or from about 1% to about 3% cationic surfactant. Cationic surfactants are useful in that they are excellent conditioning agents. In at least one embodiment, the cationic surfactant is selected from the group consisting of: behenyl trimethyl ammonium chloride (available, for example, with tradename INCROQUAT TMC-80 from Croda and ECONOL TM22 from Sanyo Kasei), distearyl dimethyl ammonium chloride (available, for example, with tradename Varisoft TA 100 from Goldschmidt), cetyl trimethyl ammonium chloride (available, for example, with tradename CA-2350 from Nikko Chemicals), hydrogenated tallow alkyl trimethyl ammonium chloride, dialkyl (14-18) dimethyl ammonium chloride, ditallow alkyl dimethyl ammonium chloride, dihydrogenated tallow alkyl dimethyl ammonium chloride, dicetyl dimethyl ammonium chloride, di(behenyl/arachidyl) dimethyl ammonium chloride, dibehenyl dimethyl ammonium chloride, stearyl dimethyl benzyl ammonium chloride, stearyl propyleneglycol phosphate dimethyl ammonium chloride, stearoyl amidopropyl dimethyl benzyl ammonium chloride, stearoyl amidopropyl dimethyl (myristylacetate) ammonium chloride, N-(stearoyl colamino formyl methy) pyridinium chloride, and mixtures thereof.

In at least one embodiment, the finishing composition comprises by weight: (i) from about 2% to about 60%, or from about 15% to about 25%, or from about 18% to about 21%, hydrophobic polyol; (ii) from about 15% to about 85%, or from about 25% to about 75%, or from about 45% to about 55%, hydrophilic polyol; (iii) from about 0.1% to about 20%, oily conditioning agent; (iv) from about 0.1% to about 10% cationic surfactant; (v) from about 5% to about 60%, inorganic heat generating agent which generates a heat by mixing with water; and (vi) from about 0.1% to about 10%, polyoxyalkylene derivative. In In at least one embodiment, the finishing composition comprises by weight: (i) from about 15% to about 22% of a hydrophobic polyol being PPG-34 (PEG-4); (ii) from about 40% to about 55% of a hydrophilic polyol being polyethylene glycol 200; (iii) from about 0.1% to about 8% of oily conditioning agents, wherein the finishing composition comprises both a dimethicone and a fatty alcohol; (iv) from about 0.1% to about 10% of a cationic surfactant being a mixture of behentrimonium chloride and cetrimonium chloride; (v) from about 5% to about 17% of an inorganic heat generating agent being anhydrous magnesium sulfate; and (vi) from about 0.1% to about 10% of a polyoxyalkylene derivative being a polyethylene/polypropylene block copolymer. In at least one embodiment, the finishing composition comprises by weight: (i) from about 15% to about 22% of a hydrophobic polyol being a polypropylene glycol; (ii) from about 40% to about 55% of a hydrophilic polyol being a polyethylene glycol; (iii) from about 0.1% to about 8% of oily conditioning agents; (iv) from about 0.1% to about 10% of a cationic surfactant; and (v) from about 0.1% to about 10% of a polyoxyalkylene derivative being a polyethylene/polypropylene block copolymer; and wherein the finishing composition is substantially free of inorganic heat generating agent.

### Optional components

A composition or formulation as described herein, or a plurality thereof, may further comprise one or more optional components known or otherwise effective for use in hair care or personal care products, provided that the optional components are physically and chemically compatible with the essential components described herein, or do not otherwise unduly impair product stability, aesthetics, or performance. Non-limiting examples of such optional components are disclosed in International Cosmetic Ingredient Dictionary, Ninth Edition, 2002, and CTFA Cosmetic Ingredient Handbook, Tenth Edition, 2004, both of which are incorporated by reference herein in their entirety. Some non-limiting examples of such optional components are disclosed below, and include plasticizer, surfactant (which may be anionic, cationic, amphoteric or nonionic), neutralizing agent, propellant, silicone compound, fatty ester, fatty alcohol, hydrocarbon comprising 14 or more carbon atoms, lanolin, vitamin, protein, preservative, dye, tint, bleach, colorant, sunscreen, gelling agent, anti-dandruff active, hair growth active, non-polymeric thickener including clay, perfume. In at least one embodiment, a composition or formulation as described herein comprises from about 0.001 % to about 10%, or 0.01 % to 5%, or 0.1% to 1% of an optional component.

### Exemplified embodiments of the first aspect

At least one embodiment relates to method of forming a polymer inside the hair shaft, the method comprising:
(a) applying an initiator composition to the hair, wherein the initiator composition comprises radical-forming agent and cosmetically acceptable carrier, wherein the initiator composition comprises from about 1% to about 4% hydrogen peroxide, and wherein the hydrogen peroxide is the sole radical-forming agent in the initiator composition; and wherein the initiator composition comprises a buffer system for stabilising the pH; then
(b) allowing the initiator composition to remain on the hair for from about 2 min to about 10 min; then
(c) applying a monomer composition to the hair, wherein the monomer composition comprises an ethylenic monomer having a molecular weight of 100 g/mole or less; then
(d) allowing the monomer composition to remain on the hair for from about 15 min to about 40 min; then
(e) applying an activating composition to the hair, wherein the activating composition comprises from about 5% to about 10% sodium thiosulfate; and wherein the activating composition has a pH of from about pH 6 to about pH 11, or from about pH 7 to about pH 9; then
(f) allowing the activating composition to remain on the hair for from about 5 min to about 15 min; then
(g) rinsing the hair; then
(h) applying to the hair a finishing composition, wherein the finishing composition comprises a hydrophobic polyol and a hydrophilic polyol;
(i) rinsing and drying the hair.

In at least one embodiment, the ethylenic monomer has a molecular weight of from about 70 g/mol to about 100 g/mole; or wherein the ethylenic monomer is selected from the group consisting of: methacrylic acid, tiglic acid, acrylic acid, 3-sulfopropyl acrylate, 3-sulfopropyl methacrylate, salts thereof, and mixtures thereof; or is acrylic acid. In at least one embodiment, the monomer composition comprises an inhibitor compound selected from the group consisting of: 2-*tert*-butyl-4-hydroxy-anisole, 3-*tert*-butyl-4-hydroxy-anisole, and mixtures thereof; or comprises the mixture of 2-*tert*-butyl-4-hydroxy-anisole and 3-*tert*-butyl-4-hydroxy-anisole.

### 2^{nd} Aspect: Process

The second aspect relates to a process for demonstrating polymerisation. The second aspect is highly related to the method of the first aspect in that it demonstrates to the consumer the mechanical benefits provided to the hair in that the observed increase in viscosity represents this improved mechanical benefit to the hair fibre. The special technical feature that the method according to the first aspect and the process of the second share is the combination of ethylenic monomer having a molecular weight of 250 g/mole or less and salt of thiosulfonic acid e.g. sodium thiosulfate. The process comprises: (i) forming a demo composition comprising: an ethylenic monomer having a molecular weight of 250 g/mole or less; an radical-forming agent; a dye; cosmetically acceptable carrier; sodium thiosulfate; wherein the demo composition exhibits a colour corresponding to the colour of the dye; (ii) and then observing an increase in viscosity and a decrease in the intensity of the colour.

In at least one embodiment, the cosmetically acceptable carrier is water. In at least one embodiment, the ethylenic monomer has a molecular weight of from about 70 g/mol to about 100 g/mole; or wherein the ethylenic monomer is selected from the group consisting of: methacrylic acid, tiglic acid, acrylic acid, 3-sulfopropyl acrylate, 3-sulfopropyl methacrylate, salts thereof, and mixtures thereof; or wherein the ethylenic monomer is acrylic acid. In at least one embodiment, the demo composition comprises from about 1% to about 20% ethylenic monomer. This may be the total amount of ethylenic monomer in the demo composition. In at least one embodiment, the radical-forming agent is a peroxide. In at least one embodiment, the demo composition comprises from about 2% to about 3% hydrogen peroxide. In at least one embodiment, the hydrogen peroxide is the sole radical-forming agent in the demo composition.. In at least one embodiment, the demo composition comprises from about 0.5% to about 20% sodium thiosulfate.

In at least one embodiment, the increase in viscosity observed in step (ii) is a solidification of the demo composition.

### 3^{rd} Aspect: Regimen

The third aspect relates to a regimen for styling hair. The regiment comprises the method according to the first aspect, wherein the method is repeated at least 2 times at a frequency of at least once every 10 days.

### 4^{th} Aspect: Kit

The fourth aspect relates to a kit. The kit comprises (i) monomer composition, wherein the monomer composition comprises an ethylenic monomer having a molecular weight of 250 g/mole or less; (ii) an activating composition, wherein the reducing composition comprises sodium thiosulfate; (iii) an initiator composition, wherein the initiator composition comprises radical-forming agent. The disclosure and description above vis-à-vis the initiator composition, activating composition and monomer composition is relevant to and combinable with the 4^{th} aspect.

In at least one embodiment, the kit comprises a formulation, wherein the formulation is selected from the group consisting of: reducing formulations, hairstyling formulations, conditioning formulations, shampoo formulations, dyeing formulations, and combinations thereof. In at least one embodiment, the kit comprises an initiator composition, wherein the initiator composition comprises from about 0.01% to about 15% peroxide. In at least one embodiment, the kit comprises a finishing composition.

### 5^{th} Aspect: Use

The third aspect relates to the use of the kit according to the fourth aspect for styling and/or treating hair. In at least one embodiment, the use is for modifying the internal region of a hair shaft. At least one embodiment relates to the use of a cosmetic composition comprising a radical-forming agent, monomer and reducing agent for forming a polymer inside the hair shaft.

### EXAMPLES

The following examples further describe and demonstrate embodiments within the scope of the present invention. The examples are given solely for the purpose of illustration and are not to be construed as limitations of the present invention.

### Composition examples

| *Monomer Composition* | Example 1 | Example 2 | Example 3 |
|---|---|---|---|
| Acrylic acid | 4.00 | - | 4.00 |
| 3-sulfopropyl acrylate | - | 10.00 | - |
| Cellosize HEC QP 4400 ¹ | 0.20 | 0.20 | 0.20 |
| EDTA | 0.12 | 0.12 | 0.12 |
| Keltrol CG-T ² | 1.00 | 1.00 | 1.00 |
| Sodium hydroxide | 0.60 | - | 1.10 |
| Phenoxethol ³ | 1.00 | 1.00 | 1.00 |
| PHB-Methylester ⁴ | 0.20 | 0.20 | 0.20 |
| Genapol® C 100 ⁵ | 0.70 | 0.70 | 0.70 |
| Cremophor EL⁶ | 0.70 | 0.70 | 0.70 |
| Fragrance | 0.30 | 0.30 | 0.30 |
| Distilled water | QSP 100% | QSP 100% | QSP 100% |
| Total | 100 | 100 | 100 |
| pH | 3.56 | 5.3 | 4.28 |

| | | | |
|---|---|---|---|
| KEY: 1 = Hydroxyethylcellulose; 2 = Xanthan gum (high molecular weight heteropolysaccharide gum produced by a pure-culture fermentation of a carbohydrate with *Xanthomonas campestris*); 3 = 2-(phenoxy)ethanol; 4 = methyl paraben; 5 = Coceth-10 (Coconut oil alcohol, ethoxylated); 6 = PEG-35 Castor Oil. | | | |

| *Initiator Composition* | Example 4 | Example 5 | Example 6 |
|---|---|---|---|
| **Phase 1** | | | |
| Purified water | 48.00 | 48.00 | 48.00 |
| Disodium phosphate | 0.08 | 0.08 | 0.08 |
| Salicylic acid | 0.10 | 0.10 | 0.10 |

| **Phase 2** | | | |
|---|---|---|---|
| Purified water | 49.96 | 47.35 | 49.56 |
| Hydrogen peroxide | 1.80 | 2.00 | 2.20 |
| Aluminium sulfate octadecahydrate | - | 2.40 | - |
| Phosphoric acid | 0.06 | 0.07 | 0.06 |
| Total | 100.00 | 100.00 | 100.00 |

To make an initiator composition as per above, phase 1 and phase 2 are created separately and then mixed together. The phase 1 components are mixed together and heated to 80°C until the salicylic acid is dissolved. Phase 1 is then stirred and cooled to 40°C. Then phase 2 is created and mixed in with phase 1 with stirring for 5 min.

| *Activating composition* | Example 7 | Example 8 |
|---|---|---|
| Demineralised water | QSP | QSP |
| 1,2-propylene glycol (double distilled) | 3.00 | 3.00 |
| Hydroxyethylcellulose ¹ | 1.00 | 1.00 |
| Polyoxyethylene (10) coconut alcohol ether (coceth-10) ² | 1.00 | 1.00 |
| PEG-35 castor oil ³ | 1.00 | 1.00 |
| Fragrance | 0.60 | 0.60 |
| Sodium thiosulphate | 5.00 | 10.00 |
| Polyquaternium-6 | 0.75 | 0.75 |
| Total | 100.00 | 100.00 |

| | | |
|---|---|---|
| KEY: ¹Natrosol 250 HHX from Hercules-Aqualon. ²Genapol C-100 from Clariant. ³Cremophor EL from BASF Corporation. | | |

| *Finishing Composition* | Example 9 | Example 10 | Example 11 |
|---|---|---|---|
| Demineralised water | QSP | - | - |
| Cetearyl alcohol ¹⁸ | 2.00 | - | - |
| Ceteareth-25 ¹⁹ | 0.50 | - | - |
| Salicylic acid | 0.10 | - | - |
| Cetyltrimethylammonium chloride ²⁰ (25%) | 0.50 | - | - |
| Polyquaternium-35 (25% in water, benzoic acid) ²¹ | 0.60 | - | - |
| Tocopheryl disodium phosphate ²² | 0.08 | - | - |
| Phosphoric acid (85%) | 0.10 | - | - |
| Hydrogen peroxide (50%) | 4.00 | - | - |
| Fragrance | 0.30 | - | - |
| Polyethylene glycol * 1 | - | QSP | QSP |
| Propyleneglycol | - | 5.0 | - |
| Polypropylene glycol *2 | - | 10.0 | 10.0 |
| Silicone oil *3 | - | - | 1.0 |
| Ester oil *4 | - | 2.0 | - |
| Anhydrous magnesium sulfate (MgSO₄) | - | 15.0 | 25.0 |
| Polyethylene/polypropylene block copolymer *5 | - | 3.0 | 3.0 |
| Cetyl Alcohol *6 | - | 1.0 | 3.6 |
| Stearyl Alcohol *7 | - | 1.8 | 6.1 |
| Behenyl trimonium chloride *8 | - | 2.0 | 2.0 |
| Distearyl dimethyl ammonium chloride *9 | - | - | 2.0 |
| Di-(alkyl carboxyethyl) hydroxyethyl methylammonium methosulfate *10 | - | 1.7 | - |
| Stearamidopropyl Dimethylamine *11 | - | - | - |
| ℓ-Glutamic acid *12 | - | - | - |
| Hydroxyethylcellulose *13 | - | - | - |
| PEG modified glyceride *14 | - | - | 5.0 |
| Perfume | - | 0.3 | 0.3 |
| Methyl paraben | - | 0.2 | 0.2 |
| Propyl paraben | - | 0.1 | 0.1 |
| 3-pyridinecarboxy acid amide | - | 0.05 | 0.05 |
| dl-Alpha tocopherol acetate | - | 0.05 | 0.05 |
| Hydrolyzed collagen *15 | - | 0.01 | 0.01 |
| Panthenol *16 | - | 0.05 | 0.05 |
| Panthenyl Ethyl Ether *17 | - | 0.05 | 0.05 |
| Octyl methoxycinnamate | - | 0.09 | 0.09 |
| Benzophenone-3 | - | 0.09 | 0.09 |
| Total | 100.00 | 100.00 | 100.00 |

| | | | |
|---|---|---|---|
| KEY: *1: Polyethylene glycol: Carbowax PEG-200 available from Union Carbide; *2: Polypropylene Glycol: PPG-34 having a tradename PP2000 available from Sanyo Kasei; *3: Silicone oil: Dimethicone having a viscosity of about 10,000 centistokes having a tradename TSF451-1MA available from GE Toshiba Silicone; *4: Ester oil: Pentaerythritol Tetraisostearate having a tradename KAK PTI available from Kokyu alcohol; *5: Polyethylene/polypropylene block copolymer: Poloxamer 338 having a tradename Newpol PE-108 available from Sanyo Chemical; *6: Cetyl Alcohol: Konol series available from Shin Nihon Rika; *7: Stearyl Alcohol: Konol series available from Shin Nihon Rika; *8 Behenyl trimonium chloride: Econol TM-22 available from Sanyo Kasei. *9: Distearyl dimethyl ammonium chloride: Varisoft TA100 available from Goldschmidt; *10: Di-(alkyl carboxyethyl) hydroxyethyl methylammonium methosulfate: Rewoquat V3620 available from Goldschmidt; *11: Stearamidopropyl Dimethylamine: SAPDMA available from Inolex; *12: ℓ-Glutamic acid: ℓ-Glutamic acid (cosmetic grade) available from Ajinomoto; *13: Hydroxyethylcellulose: Natrosol 250 MBR available from Hercules; *14: PEG modified glyceride: Tagat TO available from Goldschmidt; *15: Hydrolyzed collagen: Peptein 2000 available from Hormel; *16: Panthenol: available from Roche; *17: Panthenyl Ethyl Ether: available from Roche. ¹⁸Lanette O from Cognis; ¹⁹Cremophor A 25 from BASF Corporation; ²⁰ Detex from Cognis; ²¹Bella from Evonik; ²²Dinol from Budenheim. | | | |

### Methodology examples:

Example methods comprise the following main steps, which can be combined or order-changed to a certain degree:
- applying an initiator composition (I).
- applying a monomer composition (M).
- applying an activating composition (A).
- washing, rinsing, conditioning.

### Method examples 1 to 3:

| | **1** | **2** | **3** |
|---|---|---|---|
| **Composition 1** | I | M | M |
| **Dwell time** | 5 min | 30 min | 30 min |
| **Composition 2** | M | I | A |
| **Dwell time** | 30 min | 5 min | 5 min |
| **Composition 3** | A | A | I |
| **Dwell time** | 10 min | 10 min | 10 min |
| **Rinse** | ● | ● | ● |
| **Shampoo** | ● | ● | ● |
| **Rinse** | ● | ● | ● |
| **Condition, dry** | ● | ● | ● |

### Method examples 4 and 5 (2-steps partial combined application):

| **Method** | 4 | 5 |
|---|---|---|
| **Composition 1+2** | I+M | A+M |
| **Dwell time** | 30 min | 30 min |
| **Composition 3** | A | I |
| **Dwell time** | 10 min | 10 min |
| **Rinse** | ● | ● |
| **Shampoo** | ● | ● |
| **Rinse,** | ● | ● |
| **Condition, dry** | ● | ● |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A method of forming a polymer inside the hair shaft, the method comprising:
(a) applying an initiator composition to the hair, wherein the initiator composition comprises radical-forming agent and cosmetically acceptable carrier; then
(b) allowing the initiator composition to remain on the hair for at least 2 min; then
(c) applying a monomer composition to the hair, wherein the monomer composition comprises an ethylenic monomer having a molecular weight of 250 g/mole or less; then
(d) allowing the monomer composition to remain on the hair for at least 2 min; then
(e) applying an activating composition to the hair, wherein the activating composition comprises from about 0.5% to about 20% salt of thiosulfonic acid; then
(f) allowing the activating composition to remain on the hair for at least 2 min; then
(g) rinsing the hair.

2. A method of forming a polymer inside the hair shaft, the method comprising:
(a) applying a monomer composition to the hair, wherein the monomer composition comprises an ethylenic monomer having a molecular weight of 250 g/mole or less; then
(b) allowing the monomer composition to remain on the hair for at least 2 min; then
(c) applying an initiator composition to the hair, wherein the initiator composition comprises radical-forming agent and cosmetically acceptable carrier; then
(d) allowing the initiator composition to remain on the hair for at least 2 min; then
(e) applying an activating composition to the hair, wherein the activating composition comprises from about 0.5% to about 20% salt of thiosulfonic acid; then
(f) allowing the activating composition to remain on the hair for at least 2 min; then
(g) rinsing the hair.

3. A method of forming a polymer inside the hair shaft, the method comprising:
(a) applying a monomer composition to the hair, wherein the monomer composition comprises an ethylenic monomer having a molecular weight of 250 g/mole or less; then
(b) allowing the monomer composition to remain on the hair for at least 2 min; then
(c) applying an activating composition to the hair, wherein the activating composition comprises from about 0.5% to about 20% salt of thiosulfonic acid; then
(d) allowing the activating composition to remain on the hair for at least 2 min; then
(e) applying an initiator composition to the hair, wherein the initiator composition comprises radical-forming agent and cosmetically acceptable carrier; then
(f) allowing the initiator composition to remain on the hair for at least 2 min; then
(g) rinsing the hair.

4. A method of forming a polymer inside the hair shaft, the method comprising:
(a) applying to hair:
- an initiator composition, wherein the initiator composition comprises radical-forming agent; and
- a monomer composition to the hair, wherein the monomer composition comprises an ethylenic monomer having a molecular weight of 250 g/mole or less; and
- a cosmetically acceptable carrier;
(b) and subsequently allowing the compositions, which may be premixed before application, to remain on the hair for a period of time w, wherein time w is from about 2 min to about 45 min, or from about 3 min to about 20 min, or from about 4 min to about 10 min; then
(c) applying an activating composition to the hair, wherein the activating composition comprises from about 0.5% to about 20% salt of thiosulfonic acid; and
(d) allowing the activating composition to remain on the hair for at least 2 min;
(e) and subsequently rinsing the hair.

5. A method of forming a polymer inside the hair shaft, the method comprising:
(a) applying to hair:
- activating composition, wherein the activating composition comprises from about 0.5% to about 20% salt of thiosulfonic acid; and
- a monomer composition to the hair, wherein the monomer composition comprises an ethylenic monomer having a molecular weight of 250 g/mole or less; and
- a cosmetically acceptable carrier;
(b) and subsequently allowing the compositions, which may be premixed before application, to remain on the hair for a period of time w, wherein time w is from about 2 min to about 45 min, or from about 3 min to about 20 min, or from about 4 min to about 10 min; then
(c) applying an initiator composition to the hair, wherein the initiator composition comprises radical-forming agent; and then
(d) allowing the activating composition to remain on the hair for at least 2 min;
(e) and subsequently rinsing the hair.

6. The method of claim 1, wherein the activating composition comprises from about 8% to about 10% sodium thiosulfate, and wherein the composition is substantially free of any other reducing agent.

7. The method of any of the preceding claims, the activating composition has a pH of from about pH 6 to about pH 11, or from about pH 7 to about pH 9.

8. The method of any of the preceding claims, wherein the monomer composition comprises about 5% or less of, or is substantially free of, at least one or all of the following: reducing agent, transition metal, alcohol, ammonia, lactone compound, mercuric compound, plasticizer, surfactant, neutralizing agent, propellant, silicone compound, fatty ester, fatty alcohol, hydrocarbon comprising 14 or more carbon atoms.

9. The method of any of the preceding claims, wherein the ethylenic monomer has a molecular weight of from about 70 g/mol to about 100 g/mole; preferably wherein the ethylenic monomer is selected from the group consisting of: methacrylic acid, tiglic acid, acrylic acid, 3-sulfopropyl acrylate, 3-sulfopropyl methacrylate, salts thereof, and mixtures thereof; preferably acrylic acid.

10. The method of any of the preceding claims, wherein the monomer composition comprises an inhibitor compound selected from the group consisting of: 2-*tert*-butyl-4-hydroxyanisole, 3-*tert*-butyl-4-hydroxy-anisole, and mixtures thereof; preferably the mixture of 2-*tert*-butyl-4-hydroxy-anisole and 3-*tert*-butyl-4-hydroxy-anisole.

11. The method of any of the preceding claims, wherein the monomer composition is substantially free of any nitrogen-containing ethylenic monomers.

12. The method of any of the preceding claims, wherein the initiator composition comprises from about 2% to about 3% hydrogen peroxide, and wherein the hydrogen peroxide is the sole radical-forming agent in the initiator composition.

13. A process for demonstrating polymerisation comprising:
(i) forming a demo composition comprising:
- an ethylenic monomer having a molecular weight of 250 g/mole or less;
- an radical-forming agent;
- a dye;
- cosmetically acceptable carrier;
- sodium thiosulfate;
wherein the demo composition exhibits a colour corresponding to the colour of the dye;
(ii) and then observing an increase in viscosity and a decrease in the intensity of the colour.

14. A regimen for styling hair comprising the method according to any of claims 1 to 10, wherein the method is repeated at least 2 times at a frequency of at least once every 10 days.

15. A kit comprising:
(i) monomer composition, wherein the monomer composition comprises an ethylenic monomer having a molecular weight of 250 g/mole or less;
(ii) an activating composition, wherein the reducing composition comprises sodium thiosulfate;
(iii)an initiator composition, wherein the initiator composition comprises radical-forming agent.
